# EUROPEAN PATENT SPECIFICATION

(11) **EP 2 152 362 B1**
(45) Date of publication and mention of the grant of the patent: **08.07.2015**
(21) Application number: 07748175.2
(22) Date of filing: 28.05.2007
(51) Int. Cl.: A61N 1/37, A61N 1/365, A61B 5/0205

(54) **IMPLANTABLE MEDICAL DEVICE FOR MONITORING LUNG DEFICIENCY**
IMPLANTIERBARES MEDIZINPRODUKT ZUR ÜBERWACHUNG VON LUNGENDEFEKTEN
DISPOSITIF MÉDICAL IMPLANTABLE POUR SURVEILLER UNE INSUFFISANCE PULMONAIRE

(43) Date of publication of application: 17.02.2010
(73) Proprietor: St. Jude Medical AB, 175 84 Järfälla (SE)
(72) Inventor: BLOMQVIST, Andreas, 187 76 Täby (SE)
(74) Representative: Sjölander, Henrik
(86) International application number: PCT/SE2007/000511
(87) International publication number: WO 2008/147253

(56) References cited:
- WO-A1-01/32260
- WO-A2-2005/037220
- US-A1- 2004 133 079
- US-A1- 2005 074 741
- US-A1- 2005 142 070
- US-A1- 2006 020 295
- US-A1- 2006 195 149
- US-A1- 2006 293 604

## Description

### BACKGROUND OF THE INVENTION

### 1. Field of the invention

The present invention relates to an implantable medical device, in particular for monitoring a lung deficiency in a patient, as set out in claim 1.

The invention also concerns an implantable medical system that includes such an implantable medical device according to claim 12.

### 2. Description of the prior art

WO 2005/037220 A2 describes a device for managing respiratory disorders or symptoms by sensing and evaluating respiratory parameters, including the slopes of the inhalation and exhalation waveforms.

US2005/0142070 A1 describes an external respiratory therapy device that incorporates sensors that may be used to sense physiological conditions or parameters associated with pulmonary disease. The sensed conditions may be used to detect and/or to assess a presence of various types of pulmonary diseases. The assessment of the pulmonary disease may be utilised to control a drug therapy delivered to the patient to treat the pulmonary disease.

US 2006/0195149 A1 describes cardiac monitoring and/or stimulation methods and systems employing dyspnea measurement. An implantable cardiac device may sense transthoracic impedance and determine a patient activity level. The document describes that a system may include a lead system and a pulse generator that may incorporate one or more transthoracic impedance sensors that may be used to acquire the patient's respiration waveform, or other respiration-related information.

US 2005/0043644 A1 describes an approach for predicting disordered breathing that involves detecting one or more conditions associated with disordered breathing. The detected conditions are compared to disordered breathing prediction criteria. The document describes one method for detecting disordered breathing that involves monitoring a respiratory waveform output, for example, using a transthoracic impedance sensor. When the tidal volume of the patient's respiration, as indicated by the transthoracic impedance signal, falls below a hypopnea threshold, then a hypopnea event is declared.

US 2006/0079802 A1 describes a method and apparatus for detecting and monitoring obstructive sleep apnea. The apparatus includes an intracardiac impedance sensor to measure intracardiac impedance, a movement sensor to measure an amount of movement of a patient, and a controller operatively coupled to said intracardiac impedance sensor and said movement sensor, said controller being adapted to receive at least one of an intracardiac impedance and the amount of movement of the patient and to detect obstructive sleep apnea based upon said intracardiac impedance and said movement.

US 6,949,075 B2 describes that adventitious lung sounds indicative of lung congestion are detected using an implantable sensor. The sensor is adapted to be positioned adjacent to a pulmonary system and to output signals indicative of lung sounds in response to pulmonary system activity. A controller receives the signals and processes the signals to detect the presence of adventitious lung sounds. A respiratory cycle sensor operating in conjunction with the lung-sound sensor enables classification of an adventitious lung sound according to its time occurrence within the respiratory cycle. Posture sensing in conjunction with lung-sound sensing provides valuable additional information as to the severity of the lung congestion.

US 5,683,427 describes a heart stimulator which has a pulse generator which periodically emits stimulation pulses, at least one electrode connectable to the pulse generator and to the heart for transmitting said pulses to the heart and a respiration monitor for monitoring the respiration of the pacemaker user. The heart stimulator adapts the energy to be delivered in a stimulation pulse in response to the information acquired by the respiration monitor which indicates the current stage in the user's respiration cycle.

US 4,757,815 describes a heart pacemaker that has a pulse generator and circuitry for measuring a respiration signal of the user and a control unit for controlling the pulse generator by changing the pulse repetition rate dependent on the respiration signal. A heart action detector is provided for acquiring heart action signals and the respiration signal measuring circuitry includes detectors for measuring the amplitude fluctuations in the heart action signal and supplying those fluctuations to the control unit. It is described that the heart action signals are acquired by a heart pacemaker in a conventional manner, such as by means of a QRS detector. The amplitudes thereof are subject to fluctuations caused by the respiration cycle of the user of the heart pacemaker. The envelope for all such fluctuations corresponds to the respiration signal.

### SUMMARY OF THE INVENTION

For certain patients it is important to be able to monitor the status or progress of a lung deficiency, such as Chronic Obstructive Pulmonary Disease (COPD). It can be important to monitor such a disease or deficiency over a longer period, in order to monitor the long-term change of the lung deficiency. It may also be essential to detect sudden changes in the lung deficiency, in order to detect if the lung deficiency suddenly changes to the worse, in order to be able to take appropriate measures. The above cited documents give examples of how to monitor the respiration cycle or the like. However, the inventor of the present invention has realised that it is essential to find an improved implantable device and method for monitoring a lung deficiency, such as COPD.

One object of the present invention is therefore to provide an implantable medical device with which it is possible to monitor a lung deficiency in a correct and efficient manner. A further object is to provide such a device which has a relatively simple construction. Still an object is to provide such a device which is able to detect quite sudden changes in the lung deficiency.

The above objects are achieved by an implantable medical device for monitoring a lung deficiency in a patient according to claim 1. The device comprises a housing and a control circuit located within the housing. The control circuit is configured to be able to analyse one or more signals which represent the breathing of said patient. The control circuit is configured to be able to monitor a relationship between an expiratory phase and an inspiratory phase of the breathing cycle, and to monitor a change in said lung deficiency by monitoring a change in said relationship.

By studying said relationship it is thus possible to monitor a lung deficiency, such as COPD, in order to see how the lung deficiency progresses. For example, if the COPD becomes worse, then normally the expiratory phase of the breathing cycle becomes essentially longer, while the length of the inspiratory phase of the breathing cycle changes less. The inventor of the present invention has thus found that by monitoring said relationship, a very accurate indication of the progress of the lung deficiency is obtained.

It can be noted that a breathing cycle (BC) can be defined as an inspiratory phase (IP) followed by an expiratory phase (EP); i.e. concerning the duration of the breathing cycle BC = IP + EP. In case the patient in question were to pause the breathing during a short time interval between the expiration and the following inspiration (or between the inspiration and the expiration), then such a time interval may for example be said to belong to one of the phases or, alternatively, such an interval can be said to belong partly (e.g. 50%) to the expiratory phase and partly (e.g. 50%) to the inspiratory phase. If the patient for some reason were to pause the breathing during the inspiration (or during the expiration), then such a pause may be said to constitute part of the inspiratory phase (or the expiratory phase).

According to the invention, said relationship is a relationship between the rate of change during said expiratory phase of a signal which represents the breathing of said patient and the rate of change during the inspiratory phase of said signal. Said rate of change may simply be the derivative of the signal in question. If for example the lung deficiency becomes worse, the rate of change of said signal during the expiratory phase normally decreases more than the rate of change during the inspiratory phase. Consequently, said rates of change (or derivatives) may be used for forming the mentioned relationship.

According to a further embodiment of the device according to the invention, the device is configured to be connectable to at least one lead with at least one electrode and wherein the control circuit is configured to be able to derive, with the help of said at least one lead, the impedance over at least a portion of the patient's body, wherein the control circuit is configured to use the variation of this impedance as a signal representing the breathing of the patient. The variation of the impedance can thus be used as a signal representing the breathing. This is known from some of the documents cited above. However, it should be noted that the invention is not at all restricted to the use of impedance for detecting the breathing cycle. Any other suitable manner of detecting the breathing cycle can be used, such as a pressure sensor (for example positioned in the pericardium), the IEGM signal (see the above cited US 4,757,815) or a microphone that detects the breathing, etc.

According to a further embodiment of the device according to the invention, the device comprises at least one memory, and the control circuit is configured to carry out a procedure that includes determining said relationship and storing a corresponding value in said memory. By storing such a value in the memory, it is possible to later retrieve information of the determined relationship, and thereby of the status of the lung deficiency.

According to a further embodiment of the device according to the invention, the control circuit is configured to carry out said procedure at different occasions in time, in order to monitor if said relationship changes with time. Such occasions may for example occur each hour or once a day. This makes it possible to monitor how the lung deficiency changes over time. By carrying out the procedure quite often, it is possible to monitor quite sudden changes in the lung deficiency. By carrying out the procedure less frequently, it is possible to monitor the long-term change in the lung deficiency.

It should be noted that at each of said occasions, the relationship may, with advantage, be formed a plurality of times, and a representative value (for example the mean value of said plurality of relationships formed) can then be stored in the memory. For example, within one minute the relationship can be formed at least three times and the mean value can be stored in the memory. By forming such a representative value based on several measurements, a statistically more accurate value can be obtained.

According to a further embodiment of the device according to the invention, the device is configured to be able take into account, in addition to said relationship, also at least one further sensed property of the patient for evaluating said lung deficiency. Said further property is preferably not directly related to the signal representing the breathing pattern of the patient. By taking some further sensed property into account, a more accurate result may be obtained.

According to a further embodiment of the device according to the invention, said at least one further property is one or more properties selected from the group that comprises the hematocrit value of the blood, the oxygen level in the blood, a sound effect received from a microphone. It has been noted that when the lung deficiency, such as COPD, becomes worse, then, over a long time, the hematocrit value tends to increase. This is the body's response in order to improve the oxygen transport in the blood. Similarly, the oxygen level in the blood tends to change when the lung deficiency changes. Furthermore, with the help of a microphone a sound effect, such as a wheezing sound, related to the breathing can be detected. Such further properties therefore include information concerning the lung deficiency in question. Consequently, such further properties can be used in combination with the above described relationship in order to monitor the lung deficiency.

According to a further embodiment of the device according to the invention, the device is provided with warning means, with which the patient in whom the device is implanted, or a medical practitioner, can be alerted. It is advantageous to warn the patient or the medical practitioner when necessary. The warning to the patient may for example be a vibration of the implanted device, which vibration is sensed by the patient.

The medical practitioner can for example be alerted if the device emits a signal that is detected by a receiver outside of the patient, such that this receiver can forward a message in order to alert the medical practitioner.

According to a further embodiment of the device according to the invention, the control circuit is configured to trigger the warning means to emit an alert signal if said relationship, and possible further sensed property or properties of the patient, fulfils a predetermined criteria related to the severity of said lung deficiency. It is thus advantageous to monitor the lung deficiency and to emit an alert signal if the lung deficiency in question is severe. The patient, or the medical practitioner, can then take appropriate measures.

According to a further embodiment of the device according to the invention, the control circuit is configured to trigger the warning means if the calculated severity of said lung deficiency, based on said relationship, and possible further sensed property or properties of the patient, has changed to the worse more than a predetermined amount such that an acute attack caused by the lung deficiency is likely to be imminent. According to this embodiment, it is thus possible to warn the patient or the medical practitioner if an acute attack is imminent. A lung deficiency, such as COPD, may become worse to such an extent that the patient suffers an acute attack, which may be similar to the attacks of asthma patients. Such an attack may have severe consequences. It is therefore a particular advantage of the present invention that it is possible to predict such an attack with high accuracy. By warning the patient or the medical practitioner when such an attack is imminent, appropriate measures may be taken, such that a full-blown attack can be avoided.

According to a further embodiment of the device according to the invention, the device is configured to also be able to deliver pacing pulses to the heart of the patient in order to function as a cardiac pacemaker. It is advantageous for the device according to the invention to also be able to function as a cardiac pacemaker. Such a cardiac pacemaker may have functions that can be used also for monitoring the respiratory cycle. Consequently, such a device can be constructed to also be used for monitoring a lung deficiency as explained above. It should be noted that the device may of course also have further functions, for example in order to function as a defibrillator.

A further object of the invention is to provide an advantageous implantable medical system including a medical device according to the invention.

This object is achieved by an implantable medical system including an implantable medical device according to any one of the preceding embodiments and at least one lead connected to the device, wherein said lead comprises at least one electrode suitable for delivering pacing pulses and/or for being used when measuring, by means of the device, the impedance over at least a portion of the patients body.

With such a system, the advantages described above in connection with the device according to the invention can be obtained.

Further aspects and advantages of the present invention will become clear from the following description and claims.

### BRIEF DESCRIPTION OF THE DRAWINGS

- Fig 1: shows schematically an embodiment of an implantable medical device according to the invention connected to leads with electrodes/sensors arranged in or outside a heart.
- Fig 2: shows schematically a control circuit which may form part of the device.
- Fig 3: illustrates schematically detected respiration.
- Fig 4: illustrates schematically detected respiration similar to the one illustrated in Fig 3 but at a different occasion than the one detected in Fig 3.
- Fig 5: illustrates schematically a method

### DESCRIPTION OF PREFERRED EMBODIMENTS

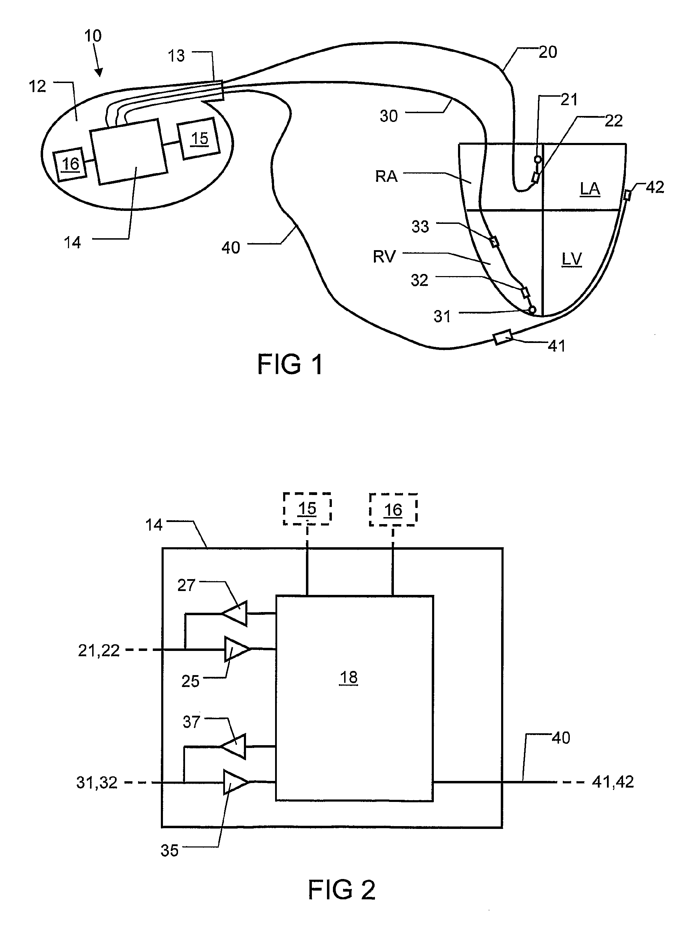

Fig 1 shows schematically an embodiment of an implantable medical device 10 according to the invention for monitoring a lung deficiency in a patient. According to the shown embodiment, the device 10 also functions as a heart stimulating device. The device 10 comprises a housing 12. The device 10 has a connector portion 13. Via the connector portion 13, the device 10 can be connected to different leads. In Fig 1 the device 10 is connected to three leads 20, 30 and 40.

Fig 1 also schematically shows a heart with a right atrium RA, a left atrium LA, a right ventricle RV and a left ventricle LV.

The lead 20 includes a pacing and sensing electrode 21, 22.

Similarly to the lead 20, the lead 30 includes a pacing and sensing electrode 31, 32. The lead 30 has a further sensor 33, which for example may be a sensor for sensing the hematocrit value of the blood or a sensor for sensing the oxygen level in the blood. Such sensors are known in the art, see, for example, PCT application PCT/SE2007/000165 and WO 00/56397.

A third lead 40 is provided with an electrode 41 and a sensor 42. The electrode 41 can for example be used in combination with some further electrode (or the housing 12) for measuring transthoracic impedance. The sensor 42 may be a microphone for detecting respiratory sounds. This microphone 42 may for example be positioned in the pericardium. The microphone 42 can be used to detect sounds caused by the breathing of the patient, for example a wheezing sound that indicates the lung deficiency in question.

In the shown example, the electrodes 21, 22, 31, 32 are bipolar electrodes with a tip portion 21,31 and a ring portion 22, 32. However, it is also possible to use unipolar electrodes. This is known to a person skilled in the art.

The device 10 comprises a control circuit 14, which will be described further below. The device 10 also comprises at least one memory 15 connected to the control circuit 14. Furthermore, the device 10 may be provided with warning means 16 with which the patient in whom the device 10 is implanted, or a medical practitioner, can be alerted. The warning means 16 may for example be a vibrator that causes the device 10 to vibrate in order to alert the patient. The warning means 16 may also be a transmitter that transmits a signal to a receiver outside of the patient, which receiver is used to alert a medical practitioner.

The device 10 together with the leads 20, 30, 40 and the pacing/sensing electrodes 21, 22, 31, 32 and the electrode/sensors 33, 41, 42 constitute a heart stimulating system according to the invention. This system can be implanted in a patient.

It can be noted that figure 1 shows a device 10 which can electrically pace and sense both an atrium and a ventricle. However, also other alternatives are possible. The device 10 can of course also constitute a monitoring device 10, which is not designed to pace the heart.

In Fig 1, the electrode 21, 22 constitutes an atrial sensing and/or pacing electrode 21, 22 which is positioned in an atrium of the heart, according to this embodiment the right atrium RA, in order to enable sensing and/or pacing of this atrium RA.

The electrode 31, 32 constitutes a ventricular sensing and pacing electrode 31, 32, which in this embodiment is positioned in the right ventricle RV. This sensing and pacing electrode 31, 32 is adapted to enable sensing and pacing of the right ventricle RV.

It is also possible that the device 10 is connected to further leads and/or further sensing/pacing electrodes, for example electrodes positioned in order to sense and/or pace the left ventricle LV and/or the left atrium LA and electrodes designed to enable defibrillation.

Fig 2 shows schematically the control circuit 14 in some more detail. The control circuit 14 includes a control portion 18 connected to the memory 15 and to the warning means 16.

The control circuit 14 includes an atrial sensing and/or pacing circuit 25, 27. In this embodiment, this circuit 25, 27 includes a sensing circuit 25 and a pacing circuit 27. The atrial sensing and/or pacing circuit 25, 27 communicates with the atrial sensing and/or pacing electrode 21, 22 via the lead 20. The atrial sensing and/or pacing circuit 25,27 is thus adapted to sense and/or pace an atrium, in this case the right atrium RA.

The control circuit 14 also includes a ventricular sensing circuit 35 and a ventricular pacing circuit 37. These circuits 35, 37 communicate with the ventricular sensing and pacing electrode 31, 32 via the lead 30. The circuits 35, 37 are thus adapted to sense and pace a ventricle, in this case the right ventricle RV.

As indicated in Fig 2, the electrode 41 and sensor 42 are also connected to the control circuit 14, via the lead 40.

The control circuit 14 is arranged to be able to operate in time cycles corresponding to heart cycles. Such an operation is normal for an implantable heart stimulating device. The time cycles are determined by preset timer intervals which also may depend on detected signals.

The control circuit 14 is also configured to be able to analyse one or more signals which represent the breathing of the patient. This can for example be done by measuring the impedance over at least a portion of the patient's body. For example a transthoracic impedance may be measured, as indicated in some of the above-cited documents. The impedance may for example be measured between the housing 12 of the device 10 and an electrode surface, such as the ring portion 32. However, many other alternatives are possible. It is for example possible to measure the impedance between the electrode 41 and another electrode 21, 22, 31, 32 or the housing 12.

The measured impedance varies with the breathing of the patient.

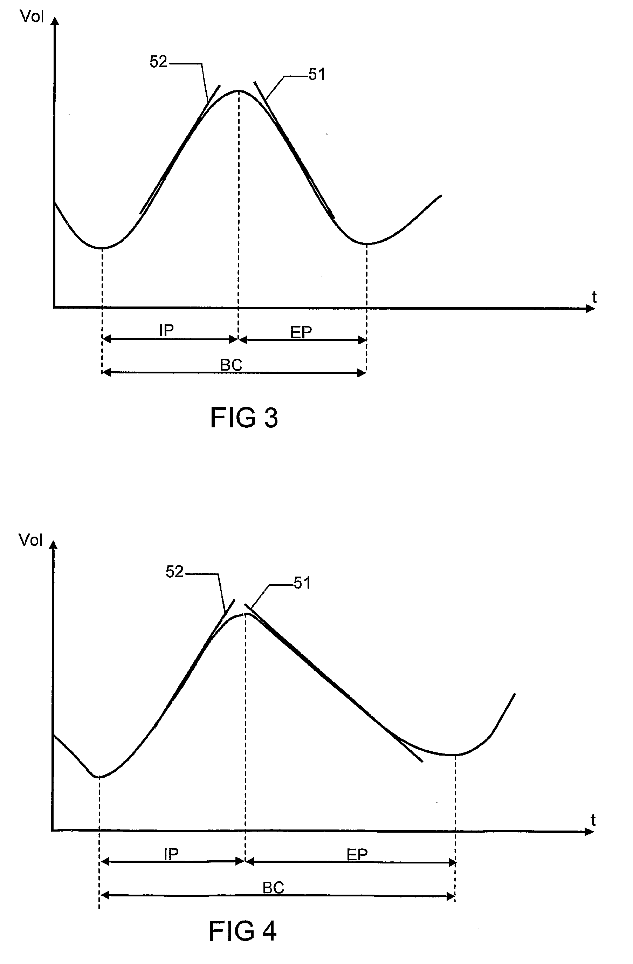

Fig. 3 illustrates the detected respiration. Vol represents the lung volume and t the time. The variation of the lung volume may thus for example be derived through the mentioned impedance measurement. In Fig. 3, BC represents a breathing cycle including an inspiratory phase IP and an expiratory phase EP.

The control circuit 14 is configured to monitor a relationship between the expiratory phase EP and the inspiratory phase IP of the breathing cycle, as explained above. The control circuit 14 is configured to monitor a change in the lung deficiency by monitoring a change in the relationship. When the lung deficiency, such as COPD, becomes worse, EP becomes longer while IP does not change as much as EP. Consequently, the ratio EP/IP increases when the COPD becomes worse.

Fig. 4 illustrates the respiration in the same manner as in Fig. 3. However, it can be seen that in Fig. 4 EP has become longer compared to in Fig. 3. Consequently, in Fig. 4 the ratio EP/IP is higher than the ratio EP/IP in Fig. 3. Fig. 4 may represent the respiration of a patient at a later occasion, than the occasion illustrated in Fig. 3. This indicates that the lung deficiency has become worse.

Fig. 3 and 4 also indicate the rate of change (or derivative) 51 of the breathing signal during the EP as well as the rate of change (or derivative) 52 of the breathing signal during the IP. The relationship between the rate of change 51 during the EP and the rate of change 52 during the IP is used as an indication of the lung deficiency. It can be seen that the absolute value of the derivative 51 is lower in Fig. 4 than in Fig. 3. The rates of change during the EP and the IP can be determined in any suitable manner. These rates of change may for example be the maximum absolute value of the derivative during the EP and IP, respectively.

The control circuit 14 is configured to carry out the procedure of determining the mentioned relationship at different occasions in time and to store a corresponding value in the memory 15. In this manner, the control circuit 14 may monitor how the relationship, and thereby the lung deficiency, changes with time.

The control circuit 14 may also be configured to take further sensed properties of the patient into account for evaluating the lung deficiency. Such properties may for example be the hematocrit value or the oxygen level of the blood, derived for example with the help of the sensor 33, or a sound effect received from the microphone 42, which represents a breathing sound of the patient.

According to this embodiment, the control circuit 14 is configured to trigger the warning means 16 to emit an alert signal if the mentioned relationship (for example the duration of EP divided by the duration of IP) has changed to the worse. The control circuit 14 may for example monitor this relationship and if the relationship changes it is possible to monitor this change and to emit an alert signal if the change in the mentioned relationship indicates that an acute attack caused by the lung deficiency is likely to be imminent. The decision whether to trigger the warning means 16 may also depend on further sensed properties, for example those described above, in order to increase the redundancy and in order to be able to determine the state of the lung deficiency with higher accuracy.

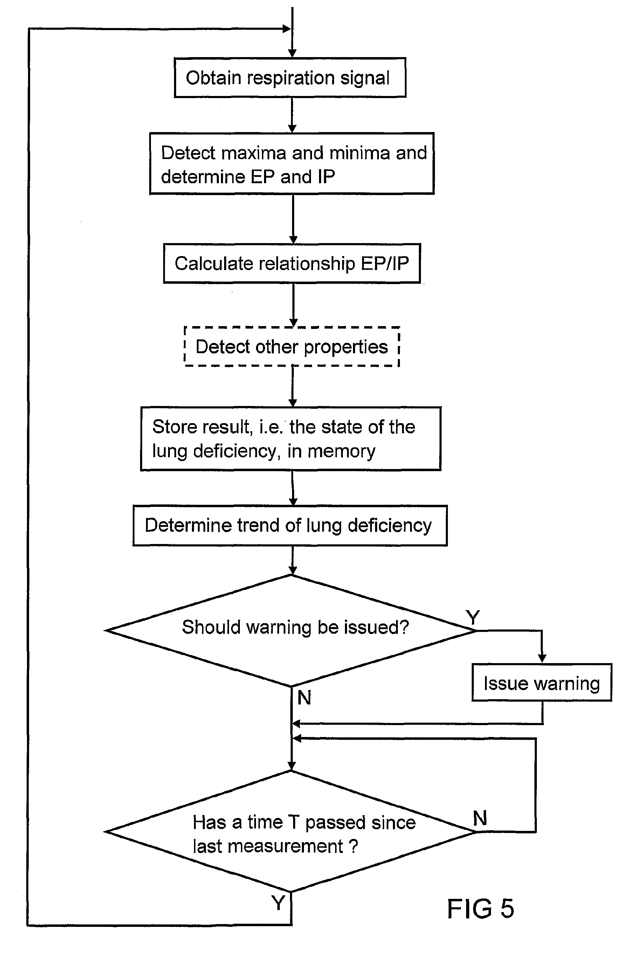

Fig. 5 illustrates schematically a method Fig. 5 can also be seen as an illustration of the operation of the device 10 according to the invention.

First (which is not shown in Fig. 5), the device 10 with the leads 20, 30, 40 and electrodes and sensors 21, 22, 31, 32, 33, 41, 42 is implanted in a patient. Then, if necessary, the device may be calibrated to the actual patient by suitable programming steps performed by the medical practitioner. Thereafter, as shown in Fig. 5, a respiration signal (for example of the kind shown in Fig. 3 and Fig. 4) is obtained. Maxima and minima in this respiration signal is detected in order to determine the expiratory phase EP and the inspiratory phase IP of a breathing cycle. At the next step, the mentioned relationship is calculated. Also other properties may be detected. Such properties may for example be the hematocrit value, the oxygen value or a lung sound as explained above.

The determined relationship and possibly also other detected properties are stored in the memory 15. The state of the lung deficiency is thus stored in the memory 15. The whole operation explained so far and shown in Fig. 5 is repeated at regular intervals, for example once an hour or once a day. However, the procedure can also be performed more frequently. For example, if the lung deficiency is severe, or has changed to the worse, then the procedure may be repeated much more frequently, for example every fifth minute.

As shown in Fig. 5, the trend of the lung deficiency is determined from the stored information. This means that it is monitored whether the mentioned relationship (and possible further detected properties) has changed such that the lung deficiency has become worse (or possibly better).

At the next step it is decided whether the patient, or the medical practitioner, should be alerted with a warning. If the trend of the lung deficiency is such that the lung deficiency has become worse such that an acute attack caused by the lung deficiency is likely to be imminent, then it is decided that the patient, or the medical practitioner, should be alerted with a warning. If this is not the case, then the procedure continues with regular intervals.

According to the method, the mentioned relationship, with which the lung deficiency can be determined, is thus monitored and if the lung deficiency becomes worse it is possible to issue a warning. However, it should be noted that the possibility of issuing a warning is an optional feature. Instead, it is of course possible to only store the monitored indications of the lung deficiency in the memory 15 such that a medical practitioner, for example with the help of so-called telemetry, may obtain information of the progress of the lung deficiency.

As mentioned above, the relationship may be another relationship than the mentioned relationship between EP and IP. For example, the monitored relationship may comprise a relationship between the rate of change during the expiratory phase of a signal which represents the breathing of said patient and rate of change during the inspiratory phase of said signal.

As also mentioned above, the method may also include the delivery of pacing pulses to the heart of the patient in order to pace the heart. Furthermore, the method, and the device, may be provided with other means which are known in connection with implantable heart monitoring and stimulating devices.

The invention is not limited to the described embodiments but may be varied and modified within the scope of the following claims.

## Claims

1. An implantable medical device (10) for monitoring a lung deficiency in a patient, said implantable medical device (10) comprising a control circuit (14) configured to i) analyse one or more signals which represent breathing of said patient, ii) monitor an expiratory phase and an inspiratory phase of a breathing cycle, and iii) monitor a change in said lung deficiency, **characterized by**:
a housing (12) within which said control circuit (14) is located, wherein said control circuit (14) is configured to i) monitor a relationship between a rate of change during an expiratory phase of a signal which represents said breathing of said patient and a rate of change during an inspiratory phase of said signal, and ii) to monitor said change in said lung deficiency by monitoring a change in said relationship.

2. An implantable medical device (10) according to claim 1, **characterized in that** said relationship comprises a relationship between a maximum absolute value of said rate of change during said expiratory phase and a maximum absolute value of said rate of change during said inspiratory phase.

3. An implantable medical device (10) according to any one of the preceding claims, **characterized in that** said implantable medical device (10) is configured to be connectable to at least one lead (20, 30, 40) with at least one electrode (21,22, 31, 32, 41) and wherein said control circuit (14) is configured to derive, with the help of said at least one lead (20, 30, 40), an impedance over at least a portion of said patient's body, wherein said control circuit (14) is configured to use a variation of this impedance as a signal representing said breathing of said patient.

4. An implantable medical device (10) according to any one of the preceding claims, **characterized in that** said implantable medical device (10) comprises at least one memory (15), and wherein said control circuit (14) is configured to carry out a procedure that includes determining said relationship and storing a corresponding value in said memory (15).

5. An implantable medical device (10) according to claim 4, **characterized in that** said control circuit (14) is configured to carry out said procedure at different occasions in time, in order to monitor if said relationship changes with time.

6. An implantable medical device (10) according to any one of the preceding claims, **characterized in that** said implantable medical device (10) is configured to take into account, in addition to said relationship, also at least one further sensed property of said patient for evaluating said lung deficiency.

7. An implantable medical device (10) according to claim 6, **characterized in that** said at least one further property is one or more properties selected from the group that comprises a hematocrit value of blood, an oxygen level in said blood, a sound effect received from a microphone (42).

8. An implantable medical device (10) according to any one of the preceding claims, **characterized in that** said implantable medical device (10) is provided with warning means (16), with which said patient in whom said implantable medical device (10) is implanted, or a medical practitioner, can be alerted.

9. An implantable medical device (10) according to claim 8, **characterized in that** said control circuit (14) is configured to trigger said warning means (16) to emit an alert signal if said relationship, and possible further sensed property or properties of said patient, fulfils a predetermined criterion related to the severity of said lung deficiency.

10. An implantable medical device (10) according to claim 9, **characterized in that** said control circuit (14) is configured to trigger said warning means (16) if a calculated severity of said lung deficiency, based on said relationship, and possible further sensed property or properties of said patient, has changed to the worse more than a predetermined amount such that an acute attack caused by said lung deficiency is likely to be imminent.

11. An implantable medical device (10) according to any one of the preceding claims, **characterized in that** said implantable medical device (10) is configured to also deliver pacing pulses to a heart of said patient in order to function as a cardiac pacemaker.

12. An implantable medical system including an implantable medical device (10) according to any one of the preceding claims and at least one lead (20, 30, 40) connected to said implantable medical device (10), wherein said lead (20, 30, 40) comprises at least one electrode (21, 22, 31, 32, 41) suitable for delivering pacing pulses and/or for being used when measuring, by means of said implantable medical device (10), an impedance over at least a portion of said patient's body.

## Patentansprüche

1. Implantierbares Medizinprodukt (10) zur Überwachung eines Lungendefekts in einem Patienten, wobei das implantierbare Medizinprodukt (10) eine Steuerschaltung (14) umfasst, die so ausgelegt ist, dass sie i) ein oder mehrere Signale analysiert, die eine Atmung des Patienten darstellen, ii) eine Ausatmungsphase und eine Einatmungsphase eines Atemzyklus überwacht und iii) eine Änderung des Lungendefekts überwacht, **gekennzeichnet durch**:
ein Gehäuse (12), in dem sich die Steuerschaltung (14) befindet, wobei die Steuerschaltung (14) so ausgelegt ist, dass sie i) eine Beziehung zwischen einer Änderungsgeschwindigkeit während einer Ausamtungsphase eines Signals, das die Atmung des Patienten darstellt, und einer Änderungsgeschwindigkeit während einer Einatmungsphase des Signals überwacht, und ii) die Änderung des Lungendefekts **durch** Überwachen einer Änderung der Beziehung überwacht.

2. Implantierbares Medizinprodukt (10) nach Anspruch 1, **dadurch gekennzeichnet, dass** die Beziehung eine Beziehung zwischen einem maximalen Absolutwert der Änderungsgeschwindigkeit während der Ausatmungsphase und einem maximalen Absolutwert der Änderungsgeschwindigkeit während der Einatmungsphase umfasst.

3. Implantierbares Medizinprodukt (10) nach einem der vorhergehenden Ansprüche, **dadurch gekennzeichnet, dass** das implantierbare Medizinprodukt (10) so ausgelegt ist, dass es mit mindestens einer Leitung (20, 30, 40) mit mindestens einer Elektrode (21, 22, 31, 32, 41) verbindbar ist und wobei die Steuerschaltung (14) so ausgelegt ist, dass sie mit Hilfe der mindestens einen Leitung (20, 30, 40) eine Impedanz über zumindest einen Abschnitt des Körpers des Patienten ableitet, wobei die Steuerschaltung (14) so ausgelegt ist, dass sie eine Veränderung dieser Impedanz als ein Signal verwendet, das die Atmung des Patienten darstellt.

4. Implantierbares Medizinprodukt (10) nach einem der vorhergehenden Ansprüche, **dadurch gekennzeichnet, dass** das implantierbare Medizinprodukt (10) mindestens einen Speicher (15) umfasst und wobei die Steuerschaltung (14) so ausgelegt ist, dass sie ein Verfahren ausführt, das ein Bestimmen der Beziehung und ein Speichern eines entsprechenden Werts in dem Speicher (15) umfasst.

5. Implantierbares Medizinprodukt (10) nach Anspruch 4, **dadurch gekennzeichnet, dass** die Steuerschaltung (14) so ausgelegt ist, dass sie das Verfahren zu unterschiedlichen Zeitpunkten ausführt, damit überwacht wird, ob sich die Beziehung mit der Zeit ändert.

6. Implantierbares Medizinprodukt (10) nach einem der vorhergehenden Ansprüche, **dadurch gekennzeichnet, dass** das implantierbare Medizinprodukt (10) so ausgelegt ist, dass es zusätzlich zu der Beziehung auch mindestens eine weitere erfasste Eigenschaft des Patienten berücksichtigt, um den Lungendefekt zu beurteilen.

7. Implantierbares Medizinprodukt (10) nach Anspruch 6, **dadurch gekennzeichnet, dass** es sich bei der mindestens einen weiteren Eigenschaft um eine oder mehrere Eigenschaften handelt, die aus der Gruppe ausgewählt ist bzw. sind, die einen Hämatokritwert des Bluts, eine Sauerstoffkonzentration in dem Blut, einen von einem Mikrofon (42) empfangenen akustischen Effekt umfasst.

8. Implantierbares Medizinprodukt (10) nach einem der vorhergehenden Ansprüche, **dadurch gekennzeichnet, dass** das implantierbare Medizinprodukt (10) mit Warnmitteln (16) versehen ist, mit denen der Patient, in dem das implantierbare Medizinprodukt (10) eingepflanzt ist, oder ein Arzt gewarnt werden kann.

9. Implantierbares Medizinprodukt (10) nach Anspruch 8, **dadurch gekennzeichnet, dass** die Steuerschaltung (14) so ausgelegt ist, dass sie die Warnmittel (16) ansteuert, damit sie ein Alarmsignal aussenden, wenn die Beziehung und (eine) mögliche weitere erfasste Eigenschaft(en) des Patienten ein vorher festgelegtes Kriterium erfüllen, das mit der Schwere des Lungendefekts zusammenhängt.

10. Implantierbares Medizinprodukt (10) nach Anspruch 9, **dadurch gekennzeichnet, dass** die Steuerschaltung (14) so ausgelegt ist, dass sie die Warnmittel (16) ansteuert, wenn sich eine berechnete Schwere des Lungendefekts, auf der Grundlage der Beziehung und einer möglichen weiteren erfassten Eigenschaft bzw. möglicher weiterer erfasster Eigenschaften des Patienten, um mehr als ein vorher festgelegtes Maß zum Schlechteren verändert hat, sodass ein durch den Lungendefekt verursachter akuter Anfall wahrscheinlich bevorsteht.

11. Implantierbares Medizinprodukt (10) nach einem der vorhergehenden Ansprüche, **dadurch gekennzeichnet, dass** das implantierbare Medizinprodukt (10) so ausgelegt ist, dass es auch Stimulationsimpulse an ein Herz des Patienten abgibt, damit es als Herzschrittmacher funktioniert.

12. Implantierbares medizinisches System, das ein implantierbares Medizinprodukt (10) nach einem der vorhergehenden Ansprüche und mindestens eine Leitung (20, 30, 40) aufweist, die mit dem implantierbaren Medizinprodukt (10) verbunden ist, wobei die Leitung (20, 30, 40) mindestens eine Elektrode (21, 22, 31, 32, 41) umfasst, die sich zum Abgeben von Stimulationsimpulsen und/oder für die Verwendung beim Messen mittels des implantierbaren Medizinprodukts (10) einer Impedanz über zumindest einen Abschnitt des Körpers des Patienten eignet.

## Revendications

1. Dispositif médical implantable (10) permettant de surveiller une insuffisance pulmonaire chez un patient, ledit dispositif médical implantable (10) comprenant un circuit de commande (14) configuré pour i) analyser un ou plusieurs signaux représentatifs de la respiration dudit patient, ii) surveiller une phase d'expiration et une phase d'inspiration d'un cycle respiratoire, et iii) surveiller un changement concernant ladite insuffisance pulmonaire, **caractérisé par** :
un boîtier (12) contenant ledit circuit de commande (14), ledit circuit de commande (14) étant configuré pour i) surveiller un rapport entre une vitesse de changement d'un signal représentatif de ladite respiration dudit patient au cours d'une phase d'expiration et une vitesse de changement dudit signal au cours d'une phase d'inspiration, et ii) surveiller ledit changement concernant ladite insuffisance pulmonaire en surveillant un changement concernant ledit rapport.

2. Dispositif médical implantable (10) selon la revendication 1, **caractérisé en ce que** ladite relation comprend un rapport entre une valeur absolue maximale de ladite vitesse du changement au cours de ladite phase d'expiration et une valeur absolue maximale de ladite vitesse du changement au cours de ladite phase d'inspiration.

3. Dispositif médical implantable (10) selon l'une quelconque des revendications précédentes, **caractérisé en ce que** ledit dispositif médical implantable (10) est configuré pour pouvoir être connecté à au moins un fil de dérivation (20, 30, 40) pourvu d'au moins une électrode (21, 22, 31, 32, 41), ledit circuit de commande (14) étant configuré pour dériver, à l'aide dudit au moins un fil de dérivation (20, 30, 40), une impédance sur au moins une partie du corps du patient, ledit circuit de commande (14) étant configuré pour exploiter une variation de cette impédance comme un signal représentatif de ladite respiration dudit patient.

4. Dispositif médical implantable (10) selon l'une quelconque des revendications précédentes, **caractérisé en ce que** ledit dispositif médical implantable (10) comprend au moins une mémoire (15), ledit circuit de commande (14) étant configuré pour effectuer une procédure comprenant la détermination de ladite relation et le stockage d'une valeur correspondante dans ladite mémoire (15).

5. Dispositif médical implantable (10) selon la revendication 4, **caractérisé en ce que** ledit circuit de commande (14) est configuré pour effectuer ladite procédure à différents moments afin de surveiller si ladite relation change avec le temps.

6. Dispositif médical implantable (10) selon l'une quelconque des revendications précédentes, **caractérisé en ce que** ledit dispositif médical implantable (10) est configuré pour prendre en compte, en sus dudit rapport, au moins une autre propriété détectée relative audit patient afin d'évaluer ladite insuffisance pulmonaire.

7. Dispositif médical implantable (10) selon la revendication 6, **caractérisé en ce que** ladite au moins une autre propriété consiste en une ou des propriétés choisies dans le groupe constitué par le taux d'hématocrite du sang, le niveau d'oxygène dans le sang et un effet sonore reçu en provenance d'un microphone (42).

8. Dispositif médical implantable (10) selon l'une quelconque des revendications précédentes, **caractérisé en ce que** ledit dispositif médical implantable (10) est pourvu d'un moyen d'avertissement (16) permettant d'alerter ledit patient chez lequel est implanté ledit dispositif médical implantable (10) ou bien un praticien.

9. Dispositif médical implantable (10) selon la revendication 8, **caractérisé en ce que** ledit circuit de commande (14) est configuré pour activer ledit moyen d'avertissement (16) afin qu'il émette un signal d'alarme si ledit rapport, et la ou les autres propriétés éventuelles détectées relatives audit patient, remplissent un critère prédéterminé associé à la sévérité de ladite insuffisance pulmonaire.

10. Dispositif médical implantable (10) selon la revendication 9, **caractérisé en ce que** ledit circuit de commande (14) est configuré pour activer ledit moyen d'avertissement (16) si la sévérité calculée de ladite insuffisance pulmonaire, fondée sur ledit rapport et sur la ou les autres propriétés éventuelles détectées relatives audit patient, s'est dégradée dans une mesure supérieure à une quantité prédéterminée si bien qu'une attaque aiguë du fait de ladite insuffisance pulmonaire est susceptible d'être imminente.

11. Dispositif médical implantable (10) selon l'une quelconque des revendications précédentes, **caractérisé en ce que** ledit dispositif médical implantable (10) est également configuré pour envoyer des impulsions de stimulation au coeur dudit patient afin d'assurer une fonction de stimulateur cardiaque.

12. Système médical implantable comportant un dispositif médical implantable (10) selon l'une quelconque des revendications précédentes et au moins un fil de dérivation (20, 30, 40) connecté audit dispositif médical implantable (10), ledit fil de dérivation (20, 30, 40) comprenant au moins une électrode (21, 22, 31, 32, 41) apte à envoyer des impulsions de stimulation et/ou destinée à servir à mesurer, au moyen dudit dispositif médical implantable (10), une impédance sur au moins une partie du corps dudit patient.
